# EUROPEAN PATENT APPLICATION

(11) **EP 4 328 292 A1**
(43) Date of publication of application: **28.02.2024**
(21) Application number: 22791736.6
(22) Date of filing: 19.04.2022
(51) Int. Cl.: C12M 1/00, C12M 1/26, C12Q 1/04, C12Q 1/24, C12Q 1/686, G01N 1/00

(54) **SPECIMEN RETENTION CARD AND SPECIMEN INSPECTION METHOD USING SAME**

(30) Priority: 20.04.2021 JP 2021070974; 07.10.2021 JP 2021165594
(71) Applicant: Universal Bio-Sampling Inc., Tokyo, 160-0003 (JP)
(72) Inventor: HIRATA, Fumiaki, Tokyo 168-0081 (JP); SHIMAGAKI, Masaaki, Otsu-shi, Shiga 520-2101 (JP)
(74) Representative: Plougmann Vingtoft a/s
(86) International application number: PCT/JP2022/018204
(87) International publication number: WO 2022/224965

(57) **Abstract**

A sample retention card is disclosed which can simplify processing of samples, identification of subjects' personal information, and the like, and also facilitates automation. The sample retention card includes: a sample carrier composed of a porous sheet and a frame surrounding the periphery of the porous sheet, the frame having an outer edge with a circular or regular polygonal shape. The porous sheet has both surfaces thereof exposed from the frame. The arrangement of the frame improves the handling of the porous sheet and eliminates the need to manually adjust the direction of the card when it is used in a testing device because the outer edge of the frame is circular or regular polygonal. The porous sheet can be punched out with a punch from each surface thereof because both surfaces of the porous sheet are exposed from the frame. This also simplifies the testing and eliminates the need to manually adjust the direction of the card when it is used in the testing device. Furthermore, this facilitates the automation.

## Description

### TECHNICAL FIELD

The present invention relates to a sample retention card and a method of testing a sample using the same.

### BACKGROUND ART

Conventionally, liquid samples, such as body fluids, e.g., blood, saliva, urine, etc., and throat swabs, are tested as they are or after being dispersed into a liquid medium such as a buffer solution. Alternatively, non-liquid samples are tested after being dispersed into a liquid medium such as a buffer solution. The place where samples are collected and the place where they are analyzed are often different. For example, samples are collected at a hospital or medical checkup center and transported to a clinical test company to conduct an analysis such as PCR in a testing room of the clinical test company. This is practiced widely. That is, samples are handled and transported in the liquid form.

Since liquid samples are inconvenient to handle during transportation, it is proposed to handle them in the solid form, instead of the liquid form, by retaining the liquid samples in a sponge sheet (Patent Document 1). During the analysis, a portion of the sponge sheet to which the sample is attached is hollowed out with a punch, etc., and the hollowed-out sponge sheet fragment is immersed in a liquid medium such as a buffer solution to disperse the sample into the medium. The resulting liquid is used for the analysis. Patent Document 1 describes that a sponge sheet made of a polyvinyl alcohol resin exhibits excellent performance as the above sponge sheet in terms of retaining samples.

### PRIOR ART PRIOR ART REFERENCES

### PATENT DOCUMENT

[PATENT DOCUMENT 1]: WO 2020/149301 A1

### SUMMARY OF THE INVENTION

### PROBLEM TO BE SOLVED BY THE INVENTION

The method described in Patent Document 1 is an excellent method that allows liquid samples to be handled as solids. However, it requires time- and labor-consuming processes such as punching out with a punch and identifying subjects' personal information. Furthermore, this method is difficult to automate.

Therefore, an object of the present invention is to provide means for enabling the sample processes and the identification of subjects' personal information and the like to be simplified, and to be easily automated. MEANS OF SOLVING THE PROBLEM

As a result of intensive research, the present inventors have found that the time and labor required for sample analysis can be reduced, while the automation can be facilitated, by arranging a frame with a circular or regular polygonal shape to surround the periphery of a sponge sheet retaining a sample, and by providing the sponge sheet in the form of a card with its both surfaces exposed from the frame, to complete the present invention.

That is, the present invention provides the following:
(1) A sample retention card comprising: a sample carrier composed of a porous sheet; and a frame surrounding the periphery of the porous sheet, the frame having an outer edge with a circular or regular polygonal shape, wherein the porous sheet has both surfaces thereof exposed from the frame.
(2) The sample retention card according to (1), wherein the porous sheet is a non-woven fabric or sponge sheet.
(3) The sample retention card according to (2), wherein the sponge sheet is composed of a polyvinyl alcohol resin.
(4) The sample retention card according to (3), wherein the polyvinyl alcohol resin is a polyvinyl formal resin.
(5) The sample retention card according to any one of (1) to (4), wherein at least one type of information selected from the group consisting of character information, a symbol, a bar code, and a QR code, which are related to the sample, is recorded on the frame, and the same information is recorded on both surfaces of the frame.
(6) The sample retention card according to any one of (1) to (4), wherein the outer edge of the frame is square.
(7) The sample retention card according to any one of (1) to (4), wherein a through hole is provided in the frame, and ridges each of which is parallel to each side of the frame are provided in a portion, other than the through hole, of at least one surface of the frame except for the through hole portion.
(8) The sample retention card according to any one of (1) to (4), wherein the porous sheet contains a surfactant that exhibits a virus inactivation ability or an agent that stabilizes a sample component.
(9) A method of testing a sample, comprising the steps of:
   providing the sample retention card according to any one of (1) to (4);
      applying a liquid sample to the porous sheet to retain the sample in the sample retention card;
   transporting the sample retention card retaining the sample to a sample analysis site; and
   detaching a portion of the porous sheet retaining the sample from the porous sheet and analyzing the sample retained in the detached porous sheet fragment.
(10) The method according to (9), further comprising the step of: drying the sample retention card between the step of retaining the sample and the step of detaching the portion of the porous sheet from the porous sheet.
(11) The method according to (9), wherein the step of detaching the porous sheet fragment from the porous sheet is performed by punching the porous sheet with a punch or hollow needle.
(12) The method according to (9), wherein the step of analyzing the sample includes a PCR method.
(13) The method according to (9), further comprising the step of disinfecting the porous sheet between the step of retaining the sample and detaching the portion of the porous sheet from the porous sheet.
(14) The method according to (13), wherein the disinfection is a virus inactivation treatment, and the virus inactivation treatment is a surfactant treatment and/or a heat treatment.
(15) Use of the card according to any one of (1) to (8) for retaining a sample.

### EFFECTS OF THE INVENTION

According to the sample retention card of the present invention, the arrangement of the frame improves the handling of the porous sheet and eliminates the need to manually adjust the direction of the card when it is used in a testing device because the outer edge of the frame is circular or regular polygonal. The porous sheet can be punched out with a punch from either surface thereof because both surfaces of the porous sheet are exposed from the frame. This also simplifies the testing and eliminates the need to manually adjust the direction of the card when it is used in the testing device. Thus, the automation is facilitated. In addition, if the same sample information is written on both surfaces of the frame, the sample information can be read from each surface. Furthermore, this facilitates the testing and eliminates the need to manually adjust the direction of the card when it is used in the testing device in the case of using the testing device.

### BRIEF DESCRIPTION OF THE DRAWINGS

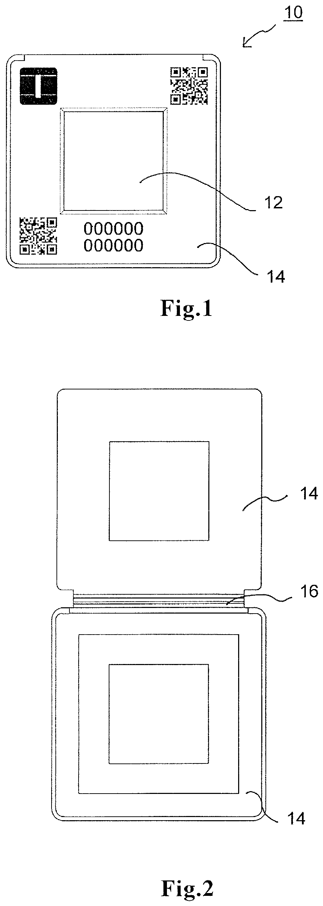

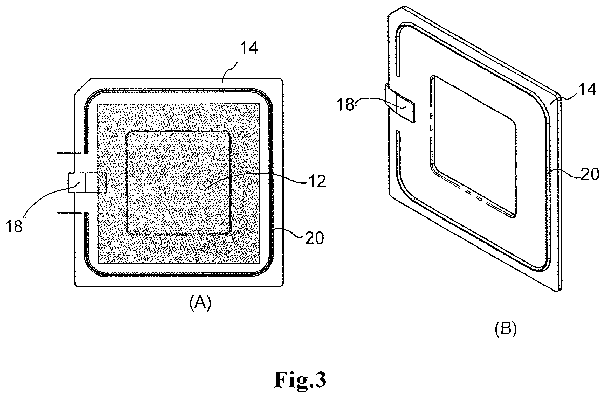

FIG. 1 is a plan view of a preferred embodiment of the sample retention card of the present invention. FIG. 2 is a plan view showing an unfolded frame in a preferred embodiment of the sample holding card of the present invention. FIGS. 3 (A) and (B) are plan and perspective views of another embodiment of the sample retention card of the present invention, respectively.

### MODE FOR CARRYING OUT THE INVENTION

A preferred embodiment of the sample retention card of the present invention will be described with reference to the accompanying drawings.

FIG. 1 is a plan view of an embodiment of the sample retention card of the present invention. A sample retention card 10 comprises a porous sheet 12. The porous sheet 12 is preferably a sponge sheet or non-woven fabric, and more preferably a sponge sheet. In particular, a sponge sheet made of a polyvinyl alcohol resin is preferable. The polyvinyl alcohol resin is a homopolymer or copolymer containing polyvinyl alcohol units. Of polyvinyl alcohol resins, a polyvinyl formal resin is particularly preferable. A sponge sheet itself made of a polyvinyl formal resin is well known and commercially available (e.g., a sponge made of a polyvinyl formal resin, manufactured by AION Co., LTD.), and commercially available products can be preferably used. It has been confirmed in Patent Document 1 that in the case of retaining a sample in a sponge made of a polyvinyl formal resin, this sample is not damaged even when dried and transported at room temperature and can provide test results equivalent to those obtained in the case of transporting a sample in the liquid form and testing it.

A frame is arranged at the periphery of the porous sheet 12, such that the frame surrounds the periphery of the porous sheet. The outer edge of the frame has a circular or regular polygonal shape in plan form (which is the shape seen from above when the frame is placed on a horizontal plane). Each vertex of a regular polygonal shape may be chamfered as shown in FIG. 1. The regular polygonal shape with chamfered vertices is also interpreted as a "regular polygonal shape". Therefore, the outer edge of the frame 14 shown in FIG. 1 is square. The porous sheet 12 has its both surfaces exposed from the frame 14. However, the shape of the window portion of the frame 14, i.e., the through portion where the porous sheet 12 is located, is not particularly limited and may be the same as or different from the shape of the outer edge of the frame 14. The area of the window portion of the frame 14 is not particularly limited, but is usually about 10% to 80%, and preferably about 15% to 60% of the area of the circular or regular polygonal shape that forms the outer edge of the frame 14. The size of the frame 14 is not particularly limited and is set as appropriate. In cases where the frame is circular, its diameter is usually about 20 mm to 100 mm, preferably about 30 mm to 90 mm, and more preferably about 40 mm to 80mm. Likewise, in cases where the frame is regular polygonal, the same goes for the diameter of a circle where the polygon is inscribed. The thickness of the porous sheet is not particularly limited and is set as appropriate, but is usually about 0.2 mm to 2 mm, and preferably about 0.3 mm to 1mm.

The material constituting the frame 14 is not particularly limited as long as it allows character information to be written therein and is robust enough to withstand handling during transportation. Examples of the material include paper and various plastics.

The frame preferably has information about the sample written therein. Examples of the information about the sample include, but are not limited to, subject's personal information (name, affiliation, address, medical condition, medical history, etc.), date and time of sample collection, collection location, etc. This information can be written in the form of character information, symbols, bar codes, QR codes (registered trademark), or the like. A plurality of pieces of such information may be written on the frame. When recording information on the frame, it is preferable to record the same information on both surfaces thereof. By recording the same information on both surfaces, the information can be read from either surface, which simplifies the operation and also facilitates the automation. In the embodiment shown in FIG. 1, two QR codes (registered trademark), letters, and symbols are written.

The sample retention card of the present invention can be formed by sandwiching the peripheral edge of the porous sheet between two frames 10. The two frames may be separately provided and arranged to sandwich the peripheral edge of the porous sheet 12. Alternatively, for example, as shown in FIG. 2, the frame 14 may be formed from one plastic sheet and folded at a hinge portion 16 to sandwich the peripheral edge of the porous sheet 12. The two frames can be glued together with an adhesive, or the two frames can be mechanically fitted and affixed together by providing concave and convex portions where these frames come into contact with each other.

To retain a sample in the sample retention card 10 of the present invention, a liquid sample is applied to the porous sheet 12. The "sample" in the present invention includes body fluids, such as blood (including whole blood, serum, and plasma), saliva, and urine, and throat swabs, etc., as well as those dispersed, suspended, or dissolved in a liquid medium such as a buffer solution. The liquid sample may be dropped onto the porous sheet 12 or applied by spraying, immersion, or the like.

The card to which the liquid sample is applied is transported to a sample analysis site (e.g., a testing room of a clinical test company). It may be dried prior to the transportation, or it may be dried naturally or with a desiccant such as silica gel before or during the transportation. It may be dried immediately before testing.

When analyzing the sample in the sample analysis site, a portion of the porous sheet is detached from the porous sheet, and the sample retained in the detached porous sheet fragment is analyzed. The operation to detach the portion of the porous sheet from the porous sheet may be performed by cutting out the portion of the porous sheet using some methods, but the cutting of the porous sheet with a punch or hollow needle (e.g., an eyelet punch, biopsy punch, hollow needle, etc.) is preferred because of its convenience. This operation can be performed manually or by an automated device.

The sample retained in the cut porous sheet fragment is then analyzed. This can usually be performed by immersing the porous sheet fragment in a liquid medium such as a buffer solution to release the test substance into the liquid medium and then analyzing the released test substance using a conventional method.

The analysis of the sample can be any well-known analysis, and a preferred example would be a PCR test for pathogens such as viruses.

In the above method, the porous sheet 12 except for the porous sheet fragment that has been detached from the porous sheet 12 remains in a state of retaining the sample. Therefore, it can be used for retesting or other tests at a later date, if necessary. For example, when a PCR test is performed in the so-called pooling method, each sample needs to be retested individually if a positive result is obtained. In such a case, the retesting can be performed using the sample retained in other portions that have not yet been detached at the first test from the porous sheet 12 of the sample retention card of the present invention. It has been confirmed that when a polyvinyl alcohol resin sponge sheet is used for the PCR test, the nucleic acid (RNA or DNA), which is the test substance retained in the sponge sheet, remains detectable by PCR for a long period of time (Patent Document 1).

Further, a step of disinfecting the porous sheet is preferably provided between the above-mentioned step of retaining the sample and the above-mentioned step of detaching the portion of the porous sheet from the porous sheet, preferably between the step of retaining the sample and the step of transporting the sample to the sample analysis site, from the viewpoint of preventing infection from the sample. The disinfection is a virus inactivation treatment, a sterilization treatment, or the like, and can be performed, for example, by a surfactant treatment or a heat treatment.

In the case of the surfactant treatment, a surfactant may be contained in the porous sheet in advance before the sample is retained. In this case, a sample retention card containing the surfactant in the porous sheet of the present invention is provided. Drying of the card in advance can provide the card that easily retains samples. In this case, the surfactant comes into contact with viruses or bacteria at a high concentration twice, i.e., when the sample is retained in the porous sheet and when it is dried, thereby making it possible to perform an inactivation treatment more effectively than the case of mixing of a surfactant solution and the sample in a liquid phase. By retaining the sample in such a card, viruses in the sample are also inactivated.

The porous sheet may also contain an agent that stabilizes a sample component. Agents that stabilize sample components include DNA and RNA degrading enzyme inhibitors, sugars (sucrose and trehalose) that inhibit protein denaturation, and preservatives (antibacterial agents). The combination of these agents is not limited. Preferably, the agent(s) is/are attached in an amount that is spread over the entire porous sheet and is then dried. Here, the concentration of the agent can be set as appropriate within the range of concentrations that achieve the stabilizing effect of a sample component. For example, RNA degradation can be inhibited by impregnating the porous sheet with about 0.008 to 0.24 units/mm 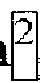 of an RNA degrading enzyme inhibitor and drying it, depending on the size of the sheet. To suppress DNA degradation, for example, chelating agents such as EDTA and agents that cleave disulfide bonds are used. In this case, preferably, the porous sheet is impregnated with about 0.1 to 0.6 µL/mm 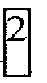 of the agent and dried. Depending on the purpose, it may be possible for a person skilled in the art to decide and execute the use of the agent in a concentration higher than this concentration. When the thickness of the porous sheet is large, it is desirable to apply more agent in order to spread it over the entire sheet. As an inhibitor of protein denaturation, it is preferable to attach a sugar such as sucrose or trehalose in order to inhibit denaturation of the dried protein. For example, preferably, the porous sheet is impregnated with a 15% to 30% aqueous solution of trehalose at about 0.2 µL/mm 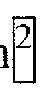 to 1.0 µL/mm 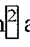 and is then dried. When the thickness of the porous sheet is large, it is desirable to apply more agent in order to spread it over the entire sheet.

Nonionic, cationic, anionic, and amphoteric surfactants can all be used as the surfactant. Surfactants with virus inactivation and bactericidal power are well known, and thus the well-known surfactants can be used. The term "virus inactivation treatment" means a treatment which causes the loss of the ability of a virus to grow *in vivo.* In examples below, as the nonionic surfactant, poly(oxyethylene) octyl phenyl ether (trade name: Triton X-100) and octyl glucoside are used, but the nonionic surfactant is not limited thereto. The surfactant treatment can also be performed by retaining the sample in the porous sheet and then applying the surfactant to the sample retained in the porous sheet. Although the surfactant may be applied only to the portion where the sample is retained, for safety reasons, it is preferable to apply the surfactant to the entire porous sheet. The surfactant treatment can be performed by various methods, such as spraying, dropping, coating, and immersion. The concentration of the surfactant is not particularly limited, but is usually about 1 to 5% by mass, preferably 2 to 3% by mass. When the surfactant is a solution, the surfactant may be applied to the porous sheet and then dried. In this case, the concentration of the surfactant is preferably 2 to 3% by mass. Even if the surfactant treatment is performed, it does not affect the analysis of PCR and the like.

A heat treatment can also be employed as a virus-killing process. After the sample is retained in the porous sheet, the virus-killing process can be easily performed by applying the heat treatment to the entire sample retention card. The conditions for the heat treatment are not particularly limited as long as the virus can be inactivated. For example, the conditions may be, 70°C to 95°C for 2 minutes to 15 minutes, preferably 80°C to 90°C for 3 minutes to 7 minutes. The heat treatment can be easily performed, for example, by heating the entire card in a water bath or oven.

As a further embodiment of the sample retention card of the present invention described above, the sample retention card shown in FIG. 3 can also be used. FIG. 3(A) is a plan view, and FIG. 3(B) is a perspective view. Ridges 20 each of which is parallel to each side of the frame 14 are provided as shown in FIG. 3. A portion where the ridge 20 is not provided is present only on one side of the frame 14, and this portion serves as a liquid sample outlet 18.

In the sample retention card of this embodiment, before drying the liquid sample, the sample retention card can be squeezed with a roller or the like, allowing the liquid sample to seep out of the porous sheet and be guided to the liquid sample outlet 18. That is, the liquid sample that have seeped out does not go out because the ridges 20 act as barriers, so that it is collected at the liquid sample outlet 18 where the ridge 20 is not present. The liquid sample collected at the liquid sample outlet 18 is dropped into a container, for example, whereby the liquid sample can be collected from the porous sheet retaining the sample. Even after drying the porous sheet, the same operation can be performed after adding a liquid such as a buffer solution to the porous sheet. Alternatively, a recess or through hole (not shown) can be provided in the frame 14 at the location of the liquid sample outlet 18 so as to serve as a liquid sample reservoir. In this case, the liquid sample stored in the recess or through hole can be collected by a pipette or the like. For the through hole, the sample can be collected from either side. After collecting the liquid sample, the sample retention card is dried if necessary, and the porous sheet is punched out with a punch or the like, as described above, so that the fragment can be used for testing.

### EXAMPLES

Hereinafter, the present invention will be specifically described based on examples. However, the present invention is not limited to the following examples. It is noted that % refers to % by mass, except for CO₂, unless otherwise specified.

### Example 1

Sample retention cards shown in FIGS. 1 and 2 were produced by using a sponge sheet made of a polyvinyl formal resin as the porous sheet. The dimension of the outer edge of the card was 54 mm square, and the size of the sponge sheet was 40 mm square and 0.5 mm thick.

### Examples 2 to 4

The sample retention cards produced in Example 1 were impregnated with various surfactants. The surfactants used were 2% Triton X-100 (trade name) (Example 2), 3% Triton X-100 (trade name) (Example 3), and octyl glucoside (Example 4), and the sponge sheet was impregnated with 650 mL of each surfactant and dried.

### Example 5 Virus Inactivation Test Using Surfactant

For the sample retention cards produced in Examples 1 to 4, a SARS-CoV-2 virus (a new type of coronavirus) solution was dropped into each card, and an experiment was conducted to confirm whether the virus was inactivated or not in each card. Details of the experiment are as follows.

### 1. Cell culture

A 96-well flat-bottom plate seeded with VeroE6/TMPRSS2 cells (purchased from JCRB Cell Bank) for virus quantification was provided as follows.
(1) After removing the culture supernatant from the T75 flask, washing was performed three times using 5 mL of PBS added.
(2) 4 mL of trypsin-EDTA solution was added, and cells were left to stand at 37°C in a CO₂ incubator until the cells were detached.
(3) Then, 8 mL of DMEM-10% FBS solution was added into a culture bottle, followed by gently pipetting, whereby the cells were collected into a 50 mL tube.
(4) The collected cells were centrifuged using a refrigerated centrifuge at 1400 rpm for 5 minutes at 4°C.
(5) The supernatant was discarded by aspiration.
(6) The resulting pellet was loosen by tapping.
(7) Then, 10 mL of DMEM-10% FBS solution was added, followed by pipetting.
(8) The cell suspension was two-fold diluted by mixing 10 µL of the cell suspension with 10 µL of a staining solution.
(9) The cell concentration was measured with a cell counter.
(10) Then, 100 µL per well of the cell suspension was added to a 96-well flat-bottom plate.
(11) The cells were cultured in a CO₂ incubator at 37°C with 5% CO₂.
(12) When the cells reached confluency, they were used for virus quantification.

### 2. Virus Inactivation Test

The virus used was a SARS-CoV-2 JPN/TY/WK-521/2020 strain (provided by the National Institute of Infectious Diseases). The stock of this virus that had been culture-amplified once with VeroE6/TMPRSS2 cells (concentration: 2.00 × 10⁷ TCID₅₀/mL) was used.
(1) The virus solution was ten-fold diluted in a DMEM-0.1% BSA solution (concentration: 2.00 × 10⁶ TCID₅₀/mL).
(2) Then, 500 µL of the diluted virus solution was added to a sponge sheet using a pipette.
(3) The sponge sheet was left to stand at room temperature for 60 minutes in a Tupperware container in which silica gel was laid.

### 3. Virus Quantification

(1) Using a pipette, 450 µL of an HBSS (Hanks' Balanced Salt Solution) was added to a 96-well flat-bottom plate.
(2) Then, 50 µL of a mixture containing the completed reaction sample and the virus solution was added to the well containing 450 µL of HBSS using a pipette, and then mixed. This operation was repeated to produce a dilution series.
(3) The culture supernatant of VeroE6/TMPRSS2 cells having reached confluency in the 96-well flat-bottom plate was removed by aspiration.
(4) Using a multi-channel pipette, 100 µL of HBSS was added.
(5) HBSS was removed by aspiration.
(6) Using a multi-channel pipette, 100 µL of HBSS was added.
(7) HBSS was removed by aspiration.
(8) Then, 100 µL of the diluted sample virus mixture was added to VeroE6/TMPRSS2 cells after HBSS was aspirated.
(9) The cells in the mixture were left to stand in a CO₂ incubator at 37°C with 5% CO₂ for 1 hour.
(10) Using a multi-channel pipette, 100 µL of HBSS was added to the plate.
(11) HBSS was removed by aspiration.
(12) Using a multi-channel pipette, 100 µL of DMEM + 0.1 w/v% BSA solution was added.
(13) The cells were cultured in a CO₂ incubator with 5% CO₂ at 37°C for 3 days.
(14) Cell degeneration was assessed using an inverted microscope.
(15) The virus concentration was calculated using the Reed-Muench method based on the number of wells with the Cytopathic Effect (CPE).

### 4. Results

The results are shown in Table 1 below. In each example, the values are averages of three runs.

**[Table 1]**

| EXAMPLES | AVERAGE VIRUS CONCENTRATION (TCID₅₀/ML) |
|---|---|
| Example 1 (no surfactant) | 38189.81 |
| Example 2 | lower than measurement limit |
| Example 3 | lower than measurement limit |
| Example 4 | lower than measurement limit |
| control (virus solution) | 82744.92 |

As shown in Table 1, it was confirmed that in all Examples 2 to 5 where the sponge sheet was impregnated with the surfactant, the virus concentration was lower than the measurement limit, which proved that the virus was inactivated.

### Example 5 Virus Inactivation Using Heat Treatment

An experiment was performed in the same way as in Example 5 using the card of Example 1. After adding the virus solution, the card was placed in a plastic bag and heated in a water bath at 85°C for 5 minutes. For comparison, a card held at room temperature was also tested in the same manner. The results are shown in Table 2 below.

**[Table 2]**

| TREATMENT | AVERAGE VIRUS CONCENTRATION (TCID₅₀/ML) |
|---|---|
| room temperature | 19996.21 |
| 85°C for 5 minutes | lower than measurement limit |
| control (virus solution) | 88854.75 |

As shown in Table 2, the virus was inactivated by the heat treatment performed at 85°C for 5 minutes.

### DESCRIPTION OF REFERENCE CHARACTERS

10 Sample retention card
12 Porous sheet
14 Frame
16 Hinge
18 Liquid sample outlet
20 Ridge

## Claims

1. A sample retention card comprising: a sample carrier composed of a porous sheet; and a frame surrounding the periphery of said porous sheet, said frame having an outer edge with a circular or regular polygonal shape, wherein said porous sheet has both surfaces thereof exposed from the frame.

2. The sample retention card according to claim 1, wherein said porous sheet is a non-woven fabric or sponge sheet.

3. The sample retention card according to claim 2, wherein said sponge sheet is composed of a polyvinyl alcohol resin.

4. The sample retention card according to claim 3, wherein said polyvinyl alcohol resin is a polyvinyl formal resin.

5. The sample retention card according to any one of claims 1 to 4, wherein at least one type of information selected from the group consisting of character information, a symbol, a bar code, and a QR code, which are related to the sample, is recorded on said frame, and the same information is recorded on both the surfaces of said frame.

6. The sample retention card according to any one of claims 1 to 4, wherein said outer edge of said frame is square.

7. The sample retention card according to any one of claims 1 to 4, wherein a through hole is provided in said frame, and ridges each of which is parallel to each side of said frame are provided in a portion, other than said through hole, of at least one surface of said frame except for the through hole portion.

8. The sample retention card according to any one of claims 1 to 4, wherein said porous sheet contains a surfactant that exhibits a virus inactivation ability or an agent that stabilizes a sample component.

9. A method of testing a sample, comprising the steps of: providing said sample retention card according to any one of claims 1 to 4;
applying a liquid sample to said porous sheet to retain said sample in said sample retention card;
transporting said sample retention card retaining said sample to a sample analysis site; and detaching a portion of said porous sheet retaining said sample from said porous sheet and analyzing said sample retained in the detached porous sheet fragment.

10. The method according to claim 9, further comprising the step of: drying said sample retention card between the step of retaining said sample and the step of detaching the portion of said porous sheet from the porous sheet.

11. The method according to claim 9, wherein the step of detaching said porous sheet fragment from said porous sheet is performed by punching said porous sheet with a punch or hollow needle.

12. The method according to claim 9, wherein the step of analyzing the sample includes a PCR method.

13. The method according to claim 9, further comprising the step of: disinfecting said porous sheet between the step of retaining said sample and detaching said portion of the porous sheet from said porous sheet.

14. The method according to claim 13, wherein the disinfection is a virus inactivation treatment, and said virus inactivation treatment is a surfactant treatment and/or a heat treatment.

15. Use of the card according to any one of claims 1 to 8 for retaining a sample.
